# FASCICULE DE BREVET EUROPEEN

(11) **EP 1 850 741 B1**
(45) Date de publication et mention de la délivrance du brevet: **05.11.2008**
(21) Numéro de dépôt: 06709173.6
(22) Date de dépôt: 26.01.2006
(51) Int. Cl.: A61B 5/022

(54) **Procédé et équipement de traitement de mesures de pression artérielle**
Verfahren und Ausrüstung zur Verarbeitung von Blutdruckmessungen
Method and equipment for processing blood pressure measurements

(30) Priorité: 26.01.2005 FR 0500785
(43) Date de publication de la demande: 07.11.2007
(73) Titulaire: A.P. Investissements A.P.J., 92800 Puteaux (FR)
(72) Inventeur: POIGNANT, Antoine, 92100 Boulogne Billancourt (FR)
(74) Mandataire: Hirsch & Associés
(86) Numéro de dépôt international: PCT/FR2006/000176
(87) Numéro de publication internationale: WO 2006/079721

(56) Documents cités:
- EP-A- 1 319 363
- US-A- 5 050 613
- US-A- 5 715 826
- US-A1- 2002 026 120
- US-A1- 2004 059 231

## Description

La présente invention concerne la mesure de la pression artérielle d'un individu.

De façon classique, la pression artérielle d'un individu se mesure en comprimant un membre par un brassard gonflable formant un garrot bloquant totalement la circulation artérielle en aval de l'artère ainsi écrasée. On dégonfle progressivement ce brassard pour mesurer sa pression, par un manomètre, lorsque le sang recommence à passer localement, c'est-à-dire que la pression de l'artère arrive à vaincre la pression externe d'écrasement exercée par le garrot. Il s'agit donc alors de la pression systolique, c'est-à-dire de la crête de pression produite par la contraction du ventricule gauche du coeur, qui vise à irriguer tout le corps.

Le bruit de passage du sang avec turbulence locale est détecté par un microphone plaqué localement sur le membre.

On maintient l'écoute pour noter de même la pression pour laquelle tout bruit de passage disparaît, c'est-à-dire que l'écoulement sanguin local est alors exempt de toute turbulence, ce qui dénote que l'artère a totalement repris sa section naturelle.

Ce type de mesure permet d'éclairer le praticien sur par exemple l'élasticité des artères, puisqu'une artère, garrottée à une certains distance du coeur, va accepter plus facilement un excès de volume sanguin si elle peut se dilater, étouffant ainsi la crête de pression systolique. Au contraire, une artère rigide n'a pas cet effet amortisseur de surpression et transmet quasi totalement le coup de boutoir du coeur jusqu'à la zone du garrot, à travers laquelle le sang ainsi sous forte pression va pouvoir plus facilement gicler en aval. Dans ce cas, la recirculation, après blocage total et relâchement progressif, intervient plus vite puisque la pression de recirculation est relativement élevée.

Les mesures ci-dessus permettant aussi de détecter une artère à section quelque peu rétrécie. Ces mesures sont en particulier intéressantes à grande distance du coeur, au niveau des chevilles, pour lesquelles la perte de charge à travers toute la longueur de l'artère considérée est susceptible d'être très élevée.

De telles mesures sont toutefois relativement fastidieuses à effectuer, de sorte que, en général, le praticien n'effectue qu'une mesure de la pression d'un bras. Il ne perçoit donc qu'imparfaitement l'état général de l'individu, c'est-à-dire qu'un traitement préventif qui serait utile risque de ne pas être prescrit.

Le document EP 1 319 363 A décrit un procédé d'aide au diagnostic pour détecter une anomalie de pression artérielle chez un individu, procédé dans lequel, lors d'un relâchement progressif d'un étranglement initial d'un membre de l'individu ayant bloqué localement toute circulation artérielle, on détecte un instant de début de recirculation artérielle pour alors effectuer une mesure d'une pression instantanée d'étranglement, l'on effectue au moins trois dites mesures, à savoir une paire de mesures, à respectivement un premier et un second bras, et une mesure à une jambe déterminée.

La présente invention vise proposer une solution tendant à diminuer l'acuité d'un tel problème.

À cet effet, l'invention concerne tout d'abord un procédé selon la revendication 1 d'aide au diagnostic pour détecter une anomalie de pression artérielle chez un individu, procédé dans lequel, lors d'un relâchement progressif d'un étranglement initial d'un membre de l'individu ayant bloqué localement toute circulation artérielle, on détecte un instant de début de recirculation artérielle pour alors effectuer une mesure d'une pression instantanée d'étranglement, procédé caractérisé par le fait que l'on effectue au moins trois dites mesures, à savoir une paire de mesures, à respectivement un premier et un second bras, et une mesure à une jambe déterminée, on compare les deux mesures de la paire pour sélectionner celle de valeur maximale, et on calcule un rapport entre la mesure relative à la dite jambe et la mesure de valeur maximale de bras, pour ainsi obtenir un rapport de pressions artérielles systoliques.

La valeur la plus élevée de pression parmi celles des deux bras fournit ainsi une référence de pression qui présente une pertinence optimale, puisqu'elle correspond à un endroit de prise de pression pour lequel la perte de charge, depuis le coeur, se trouve être minimale. L'interprétation de la mesure relative à la jambe est donc améliorée- Le dit rapport peut représenter le résultat de la division de la pression de jambe par celle de bras, ou bien l'inverse. Si la dite jambe déterminée est une première jambe examinée et qu'on effectue en outre une dite mesure à une seconde jambe, on peut comparer mutuellement les deux mesures des jambes pour déterminer un écart entre les deux rapports respectifs de pressions systoliques.

Avantageusement, on déroule une séquence de cycles élémentaires comportant, chacun pour un membre qui lui est particulier, l'étranglement et le relâchement correspondant, on mémorise à chaque fois la mesure de pression respective, et ensuite on relit les mesures de pression pour calculer le dit rapport de pressions artérielles systoliques. L'intérêt de commander un tel fonctionnement séquentiel de divers brassards ou équivalents de mesure de pression réside dans le fait que, à tout instant, un seul membre est comprimé, de sorte que la compression de l'un ne peut pas fausser les mesures des autres. En d'autres termes, le sang provenant du coeur accède encore aux artères sur toute leur longueur, hormis le tronçon aval de celle en cours de mesure.

Avantageusement, on émet les mesures sur un réseau de transmission de données à destination d'une unité centrale effectuant le dit calcul, l'unité centrale distante pouvant même télécommander l'exécution des mesures.

On utilise avantageusement des circuits d'un téléphone portable pour assurer certaines fonctions concernant les dites mesures, par exemple la détection de circulation artérielle par le microphone téléphonique.

L'invention concerne aussi un équipement selon la revendication 3 pour la mise en oeuvre du procédé selon l'invention, comportant une unité centrale reliée à des moyens d'interface agencés pour recevoir en entrée et fournir à l'unité centrale une pluralité d'au moins trois signaux de pression artérielle, l'unité centrale comprenant des moyens comparateurs reliés aux moyens d'interface et agencés pour comparer mutuellement deux signaux d'une paire constituée par des premier et deuxième dits signaux de pression, et sélectionner une valeur maximale de pression dans la dite paire de signaux de pression, et des moyens de calcul, reliés en aval des moyens d'interface et en sortie des moyens comparateurs, agencés pour calculer un premier rapport de pressions artérielles systoliques entre une valeur d'un dit signal de pression, troisième, et la dite valeur maximale de pression sélectionnée dans la paire.

On notera que l'unité centrale peut être prévue pour recevoir les valeurs pertinentes de pression limite de recirculation, en provenance de brassards de prise de pression comportant un microphone de détection de recirculation commandant une logique appropriée de mémorisation de la valeur de pression alors courante. En pareil cas, le travail essentiel de l'unité centrale peut se limiter aux moyens d'établissement du dit rapport. On peut toutefois considérer qu'une telle unité centrale, à tâche limitée, et un tel ensemble de brassards comportant de l'intelligence, et entre autres des moyens de mémorisation, constitue une forme de réalisation particulière de l'invention, c'est-à-dire sous forme répartie.

Les moyens d'interface peuvent comporter une pieuvre de fils de liaison avec les divers brassards, reliés ainsi aux circuits d'interface de façon fixe ou bien amovible, avec donc des connecteurs à au moins une extrémité de chaque liaison.

Les moyens d'interface sont de préférence agencés pour recevoir et fournir aussi au moins un quatrième signal de pression artérielle et les moyens de calcul sont agencés pour calculer un deuxième rapport de pressions artérielles systoliques entre la valeur du quatrième signal de pression et la dite valeur maximale de pression sélectionnée dans la paire.

Les moyens de calcul peuvent être agencés pour effectuer un calcul d'écart, absolu ou relatif, entre les premier et deuxième rapports de pressions artérielles systoliques.

Avantageusement, les moyens d'interface comportent des circuits de multiplexage agencés pour scruter successivement une dite pluralité de capteurs de circulation sanguine.

Des circuits séquenceurs peuvent en particulier être agencés pour commander une succession de cycles élémentaires, d'étranglement initial et de relâchement de l'étranglement de membre, par respectivement une dite pluralité d'actionneurs prévus pour provoquer les étranglements respectifs.

Les moyens d'interface peuvent être agencés pour fournir les signaux de pression de façon continue en phase d'étranglement et l'unité centrale comporte alors des moyens de comparaison, reliés aux moyens d'interface, agencés pour comparer les signaux de pression à une valeur de seuil maximal et pour, en cas de dépassement du seuil, engendrer des signaux respectifs, d'inhibition de commande de moyens d'étranglement de membre, et les transmettre aux moyens d'interface, agencés pour les retransmettre en sortie de l'équipement. On peut ainsi ouvrir une soupape d'écrêtage de pression. On notera que, si une pompe de mise en pression d'un brassard est aussi commandée par l'équipement, celui-ci peut émettre, à destination de celle-ci, un ordre d'arrêt.

De même, les moyens d'interface peuvent être agencés pour fournir les signaux de pression de façon continue, en phase de relâchement de chaque étranglement de membre, ainsi qu'une dite pluralité de signaux associés de capteur sonore représentant un bruit de circulation artérielle, et l'unité centrale comporte des moyens d'analyse sonore agencés pour comparer chaque signal de capteur sonore à un seuil minimal et pour, en cas de dépassement du seuil, commander une mémorisation d'une valeur instantanée du signal de pression associé et commander ultérieurement l'activation des moyens comparateurs et des moyens de calcul sur la base de la dite valeur de pression mémorisée.

Les moyens d'analyse sonore sont avantageusement agencés pour effectuer une analyse spectrale des signaux de capteur sonore et pour en comparer un résultat à un modèle afin d'en déduire une différence mutuelle et de la comparer à un seuil minimal pour décider si un bruit capté représente une dite recirculation artérielle.

Les moyens d'interface peuvent comporter des moyens de numérisation de dits signaux, analogiques, de capteurs sonores.

Commodément, il peut être prévu des moyens de relations homme-machine agencés pour au moins restituer la valeur du premier rapport de pressions artérielles systoliques, et aussi, de préférence, les valeurs de pression des membres. Les moyens de relations homme-machine, par exemple un afficheur ou encore un synthétiseur vocal, sont avantageusement agencés pour exécuter une séquence de cycles comportant, chacun, des restitutions successives du premier rapport de pressions artérielles systoliques et des pressions des membres.

L'unité centrale est avantageusement agencée pour traiter concurremment plusieurs dites pluralités d'au moins trois signaux de pression artérielle, pour respectivement plusieurs individus et fonctionne alors de préférence selon des tranches temporelles entrelacées allouées successivement aux traitements respectifs des dites pluralités de signaux.

L'unité centrale peut en particulier être agencée pour effectuer une scrutation cyclique de requêtes de traitement associées à de dites pluralités de signaux à traiter.

Les moyens d'interface peuvent comporter des circuits de liaison réseau prévus pour être reliés à un réseau de transmission de données, local ou international, l'unité centrale pouvant alors être agencée pour transmettre, aux circuits de liaison réseau, des informations contenant le dit premier rapport de pressions artérielles systoliques. Un serveur formant base de données centralisée peut ainsi recueillir les résultats des mesures pour tenir à jour les dossiers médicaux d'un grand nombre d'individus.

L'unité centrale peut aussi être agencée pour recevoir, des circuits de liaison réseau, les dits premier, deuxième et troisième signaux de pression.

L'unité centrale peut en particulier être agencée pour transmettre, aux circuits de liaison réseau, des ordres de télécommande d'organes de tensiomètres de fourniture des dits premier, deuxième et troisième signaux de pression.

Dans une forme de réalisation, les circuits de liaison réseau comportent des circuits de niveau 3, dans la classification OSI, agencés pour échanger, avec le réseau, des messages d'établissement de communication avec un correspondant et des circuits de niveau 2 agencés pour, une fois la communication établie, relayer, avec adaptation de format, des échanges de données liées à l'application considérée, d'aide au diagnostic par mesure de pression artérielle, entre le dit équipement et le correspondant.

L'équipement peut comprendre le dit réseau de transmission de données et au moins un auto-tensiomètre comportant un moyen de détermination de détection de circulation artérielle et un moyen de détermination de valeurs de mesure de pression artérielle relié en sortie à des circuits de raccordement au dit réseau, agencés pour recevoir les mesures de pression artérielle et les transmettre sur le réseau.

L'auto-tensiomètre comporte avantageusement une unité centrale locale avec un bloc de calcul et des moyens mémoires associés contenant des instructions de détermination de valeurs de mesure de pression artérielle et des instructions de détection de circulation artérielle, pour constituer, avec le bloc de calcul, le dit moyen de détermination de valeurs de mesure de pression artérielle et le dit moyen de détermination de détection de circulation artérielle.

Les moyens mémoires sont de préférence agencés pour recevoir les dites instructions à travers les circuits de raccordement réseau. On peut ainsi effectuer la mise en service et le maintien à niveau du logiciel considéré.

L'unité centrale locale est de préférence agencée pour recevoir, à travers les circuits de raccordement, des commandes de lancement d'au moins trois cycles élémentaires correspondant respectivement aux dites mesures de pression.

Avantageusement, les circuits de raccordement et de préférence aussi le bloc de calcul sont intégrés dans le poste téléphonique et ce dernier peut être prévu pour fonctionner en partage de temps entre des tâches de gestion de liaison téléphonique et des tâches liées au fonctionnement de l'équipement. Le coût des fonctions spécifiques à l'équipement est ainsi marginal.

Commodément, les circuits de raccordement sont prévus pour un raccordement avec un réseau de radiotéléphonie, c'est-à-dire qu'un téléphone portable peut contenir toutes les fonctions logiques nécessaires à la gestion du brassard. Il peut même être prévu que tout ou partie des organes capteurs ou actionneurs du brassard soit intégré dans le téléphone portable, alors équipé d'une enveloppe gonflable, un circuit de microphone du poste téléphonique étant agencé pour être relié à des circuits de détection de circulation artérielle, commandant la mémorisation d'une mesure de pression.

L'équipement selon l'invention, et donc en particulier les moyens de calcul, peut être intégré dans un téléphone portable. L'utilisateur dispose ainsi des moyens voulus pour la mise en oeuvre du procédé de l'invention, éventuellement sur commande externe provenant du réseau téléphonique, avec transmission éventuelle des résultats vers un centre de surveillance.

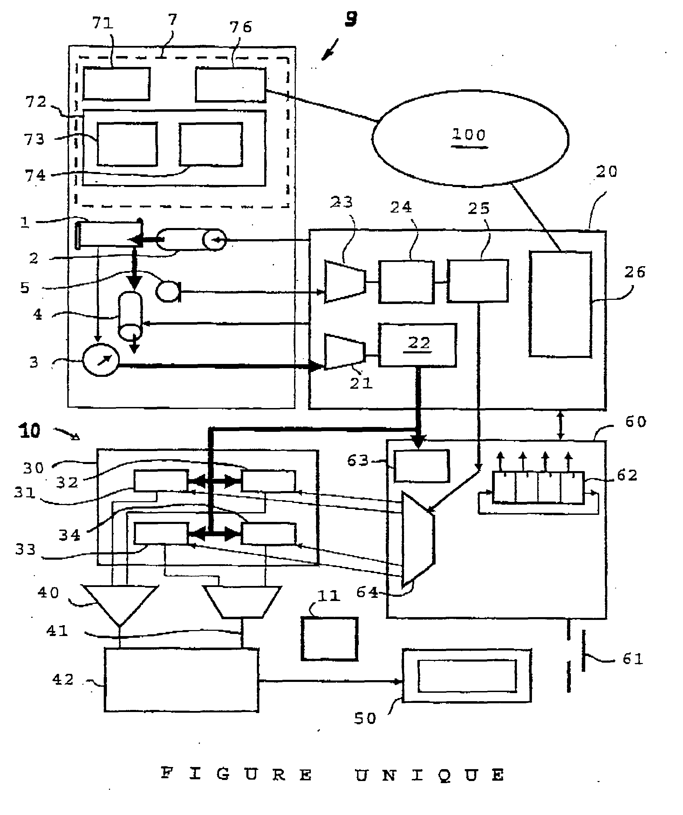

La présente invention sera mieux comprise à l'aide de la description suivante d'un mode de mise en oeuvre du procédé de l'invention et d'un équipement mettant en oeuvre le dit procédé, en référence à la figure unique qui est un diagramme par blocs fonctionnels de l'équipement.

L'équipement représenté sur la figure comporte une unité centrale 10 reliée à des circuits d'interface 20 agencés pour recevoir en entrée, et fournir à l'unité centrale 10, une pluralité d'au moins trois, ici quatre, signaux de pression artérielle, l'unité centrale 10 comprenant des circuits comparateurs 40 reliés aux circuits d'interface 20 afin de comparer mutuellement deux signaux d'une paire constituée par des premier et deuxième dits signaux de pression, et sélectionner une valeur maximale de pression dans la dite paire de signaux de pression, et comprenant des circuits de calcul 42, reliés en aval des circuits d'interface 20 et en sortie des circuits de comparaison 40, agencés pour calculer un premier rapport de pressions artérielles systoliques entre une valeur d'un dit signal de pression, troisième, et la dite valeur maximale de pression sélectionnée dans la paire.

L'équipement ci-dessus est relié à une pluralité d'ici quatre brassards 9 de prise de tension aux quatre membres respectifs d'un individu, un seul des brassards 9 étant représenté. Les brassards 9 peuvent être uniquement analogiques, ou comporter des circuits numériques pour ainsi constituer des auto-tensiomètres numérisant les mesures et effectuer des calculs numériques sur les pressions mesurées.

Un tel brassard 9 comporte une enveloppe gonflable 1 en forme de bande portant, à deux extrémités respectives, deux éléments de fixation mutuelle, tels qu'au moins un crochet et un oeillet ou encore deux bandes Velcro, afin de constituer un collier, de longueur fixe une fois posé, entourant le membre considéré. Une pompe 2 permet de gonfler un volume interne de l'enveloppe 1 au-delà d'une pression bloquant toute circulation artérielle et un détecteur de pression, c'est-à-dire un manomètre 3, relié au volume interne de l'enveloppe 1, fournit une valeur de pression instantanée. Un détendeur 4, de mise progressive à l'évent, introduit une fuite contrôlée de l'air du volume interne, pour ainsi en ramener graduellement la pression jusqu'à la valeur de pression atmosphérique. Le détendeur 4 peut être un élément passif, dans la mesure où la fuite qu'il induit est limitée par rapport à l'apport d'air de la pompe 2, ou bien le détendeur 4 peut comporter un actionneur commandant une soupape d'ouverture d'un passage de mise à l'évent, de section limitée. Un microphone 5 est porté par une face dite interne de l'enveloppe 1, c'est-à-dire tournée vers l'intérieur du fourreau que constitue le brassard 9, et donc plaquée contre la peau de l'individu.

La référence 7 désigne une unité centrale optionnelle d'auto-tensiomètre, comportant un bloc de calcul 71, ici un microprocesseur, une mémoire 72 d'instructions pour commander le bloc de calcul 71, et des circuits 76 de raccordement à un réseau de transmission de données 100. Dans un premier temps, il ne sera pas fait appel à l'unité centrale 7 pour expliquer le fonctionnement de l'équipement.

L'unité centrale 10 comporte une base de temps 11 fournissant des signaux d'horloge à diverses fréquences voulues pour les divers blocs fonctionnels.

Les circuits d'interface 20 comportent, dans cet exemple, un multiplexeur d'entrée de signaux de pression 21 à quatre voies d'entrée respectivement reliées en sortie des quatre manomètres 3 pour ainsi en recevoir les signaux respectifs de pression instantanée, ici sous forme analogique. Un séquenceur 60, à bouton d'activation 61, commande le multiplexeur 21, pour scruter successivement chacune des pressions des divers manomètres 3. Le multiplexeur 21 est relié en sortie à un circuit convertisseur analogique / numérique 22 dont les valeurs numériques de pression ainsi obtenues sont aiguillées, par le séquenceur 60, vers une mémoire particulière parmi quatre mémoires 31, 32, 33 et 34 appartenant à des circuits mémoires 30, respectivement réservées, dans cet ordre, à la mesure de pression du bras gauche, du bras droit, de la jambe gauche et de la jambe droite.

Les mémoires de pression de bras 31 et 32 sont accessibles en lecture aux circuits comparateurs 40, qui en sélectionnent ainsi celle représentant la pression de bras maximale, la plus élevée des deux, pour ensuite la transmettre aux circuits de calcul 42, reliés en lecture aux mémoires de pression de jambe 33 et 34. Un premier calcul consiste à diviser la valeur de pression de la mémoire de jambe gauche 33 par la valeur de pression de bras maximale pour ainsi obtenir un rapport de pressions artérielles systoliques de la jambe considérée, ici gauche.

Dans cette forme de réalisation, il est aussi prévu d'effectuer un calcul du même genre pour la jambe de droite, c'est-à-dire en prenant en compte la valeur de pression de la mémoire 34. Un multiplexeur 41, commandé par le séquenceur 60, est ainsi relié en entrée aux deux sorties des mémoires de jambe respectives 33 et 34, et relié en sortie à une entrée de valeur des circuits de calcul 42 .

Un afficheur 50 reçoit successivement deux nombres représentant les valeurs des deux rapports ci-dessus et les affiche ainsi en séquence- L'afficheur 50 peut en outre, de façon ici non représentée, être relié aux sorties des mémoires 31, 32, 33, 34 pour aussi en afficher automatiquement et séquentiellement les valeurs respectives en plus des deux rapports ci-dessus. L'affichage peut être numérique mais il peut aussi être, ou comporter, un affichage analogique, par exemple du genre à barre de diodes électroluminescentes ou de pavés élémentaires de cristaux liquides, LCD, affichage dans lequel on excite un nombre plus ou moins grand de pavés successifs pour présenter un segment dont la longueur varie comme la grandeur de la valeur affichée. Il peut aussi être prévu un fond d'affichage dont la couleur évolue selon la valeur affichée, en passant par exemple du vert au rouge en cas de résultat médicalement mauvais.

Des circuits d'échange de signaux de mesure, autres que les signaux de pression ci-dessus, ou de commande entre l'équipement et les divers brassards 9, vont maintenant être décrits.

Les circuits d'interface 20 comportent un multiplexeur 23 d'entrée de signaux sonores, commandé par le séquenceur 60 et comportant quatre voies d'entrée respectivement reliées en sortie des microphones 5 respectifs. Un convertisseur analogique / numérique 24, relié en sortie du multiplexeur 23, fournit des signaux numériques correspondants à des circuits d'analyse sonore 25, agencés pour comparer le signal de chaque microphone 5 à un seuil minimal et pour, en cas de dépassement du seuil, envoyer une commande de mémorisation de pression courante à un démultiplexeur 64 des circuits séquenceurs 60, qui l'aiguille vers la mémoire 31 à 34 voulue. Il est ainsi effectué une mémorisation d'une valeur instantanée du signal de pression associé au bruit capté et cette commande est elle-même mémorisée pour ultérieurement provoquer l'activation des circuits comparateurs 40 et des circuits de calcul 42 sur la base de la dite valeur de pression mémorisée. Dans ce but de mémorisation, la valeur instantanée, c'est-à-dire courante, de la pression du manomètre 3 considéré est appliquée en entrée de la mémoire 31 à 34 correspondante.

La commande ci-dessus de mémorisation de pression est transmise par les circuits d'analyse sonore 25 aux circuits séquenceurs 60, qui émettent en conséquence une impulsion d'horloge de mémorisation (flèches) vers celle des mémoires 31 à 34 qui est concernée.

En variante, et de façon duale, au lieu d'empêcher la mémorisation d'une suite de valeurs décroissantes de pression supérieures, donc sans intérêt, à la pression limite d'étranglement, il peut au contraire être prévu de les mémoriser temporairement l'une après l'autre, chaque valeur nouvelle venant écraser la précédente en mémoire, et d'arrêter une telle mise à jour de la mémoire 31 à 34 considérée lorsque la recirculation artérielle est détectée par les circuits d'analyse sonore 25. Les circuits séquenceurs 60 émettent donc cycliquement de telles impulsions d'horloge pendant la phase initiale correspondant à la plage de pressions excessives, comprises entre une pression initiale maximale, de blocage de circulation artérielle, et la pression limite, de recirculation. L'arrêt ci-dessus s'effectue par cessation d'envoi des impulsions d'horloge de mémorisation commandant les mémoires 31 à 34.

Les circuits d'analyse sonore 25 sont ici agencés pour effectuer une analyse spectrale, donc fréquentielle, des signaux de microphone 5 et pour en comparer un résultat à un modèle afin d'en déduire une différence mutuelle et de la comparer à un seuil minimal pour décider si un bruit capté représente une dite recirculation artérielle. Cette analyse fréquentielle porte ici sur une analyse par transformée de Fourier discrète, fournissant cycliquement, à intervalles de temps successifs, un spectre représentant des amplitudes de diverses composantes fréquentielles. Le spectre, mis à jour à chaque intervalle de temps, est comparé à un dit modèle de spectre pour, d'après un écart représentant leur différence mutuelle en ce qui concerne chaque raie fréquentielle, décider si une circulation artérielle est perçue.

Le gonflage des diverses enveloppes 1 par les pompes 2 respectives s'effectue sous la commande des circuits séquenceurs 60. A cet effet, les circuits séquenceurs 60 comportent un registre à décalage 62 série / parallèle à huit étages binaires, dont, à l'état de repos, une entrée de valeur est polarisée à un niveau binaire, ici le bit de valeur "1" , représentant une commande d'activation de pompe 2. Le bit "1" ci-dessus provient, dans cet exemple, de la sortie du huitième étage, les sept autres étages, de rang 1 à 7, contenant la valeur opposée, "0". Il s'agit donc d'un anneau logique dans lequel peut tourner le "1". Les quatre étages de rang impair sont reliés en sortie aux quatre pompes 2 respectives, pour les activer, et désactiver, l'une après l'autre, par le bit "1".

Pour commander le gonflage d'une première des enveloppes 1, le séquenceur 60 émet une impulsion d'horloge décalant d'une position le contenu du registre 62, de sorte que le "1" du huitième étage, "poussé" par le "0" du septième étage qui le remplace, passe, en rebouclage, dans le premier étage, qui est relié à une entrée d'activation de l'une des pompes 2, à travers les circuits d'interface 20. La pression instantanée de l'enveloppe 1 considérée est alors surveillée, en sortie du convertisseur 22, par des circuits de surveillance 63 appartenant aux circuits séquenceurs 60, et est comparée à un seuil de pression maximale, c'est-à-dire de consigne de pression de gonflage. Lorsque le seuil maximal est franchi à la hausse, les circuits de surveillance 63 commandent l'émission d'une deuxième impulsion d'avance du registre 62, de sorte que le "1" de son premier étage, passant dans le deuxième étage, est remplacé par le "0" en sortie du huitième étage. La pompe 2 considérée s'arrête donc. Si le détendeur 4 est de type actif, une commande d'ouverture lui est envoyée à travers une liaison non représentée des circuits d'interface 20.

La pression initiale étant ainsi établie, le cycle de mesure de pression du brassard 9 considéré se poursuit par la phase de détente ainsi amorcée, avec la surveillance de la pression instantanée exposée plus haut. Lorsque la pression limite de recirculation artérielle est détectée et mémorisée, un deuxième même cycle est lancé par envoi d'une troisième impulsion d'horloge, qui fait passer le "1" d'activation dans le troisième étage du registre 62. Le cycle exposé ci-dessus se répète ainsi, mais pour une autre des quatre pompes 2.

Dans les circuits d'interface 20, la référence 26 désigne des circuits de liaison avec le réseau de transmission de données 100, ici le réseau Internet. L'équipement peut ainsi échanger aussi des signaux d'interface, des types exposés ci-dessus, avec des brassards 9 ou auto-tensiomètres distants munis de circuits d'interface Internet, tels que les circuits de liaison ou raccordement 26 ou 76.

L'unité centrale 10 est en outre agencée pour traiter concurremment plusieurs dites pluralités d'au moins trois, quatre dans cet exemple, signaux de pression artérielle, pour respectivement plusieurs individus. Les circuits d'interface 20 représentés sont donc dupliqués de façon correspondante ou bien, de préférence, la capacité des blocs fonctionnels est accrue. Il en est de même pour les blocs fonctionnels de l'unité centrale 10.

Les circuits de liaison réseau 26 comportent, selon la classification OSI, des circuits d'interface physique avec l'Internet 100, de niveau 1, des circuits de gestion de format et de débit de messages d'informations échangés avec l'Internet, niveau 2, et des circuits de gestion d'échange de messages de service pour établir et rompre les liaisons Internet, niveau 3- La communication est ainsi établie avec un équipement distant comportant par exemple des auto-tensiomètres équipés de circuits d'interface du même genre que les circuits de liaison réseau 26. L'application particulière ici considérée, c'est-à-dire la mesure à distance des pressions artérielles et éventuellement la gestion des pompes 2 ou actionneurs équivalents et des capteurs de circulation associés 5, peut alors être exécutée au moyen d'un logiciel applicatif logé dans une mémoire de l'unité centrale 10. Comme évoqué ci-dessus, le logiciel applicatif effectue la scrutation cyclique des mesures des auto-tensiomètres distants, voire aussi locaux, et leur transmet les télécommandes éventuelles des pompes 2 et capteurs sonores 5 et autres. Les mesures et les rapports calculés de pression peuvent en outre être transmis depuis l'unité centrale 10, par le réseau Internet 100, à un équipement distant d'exploitation des résultats, par exemple un hôpital ou encore un serveur de tenue à jour centralisée de fichiers de suivi médical des individus.

Chaque pluralité d'ici quatre brassards 9 ou auto-tensiomètres est associée à un identifiant permettant de retrouver en mémoire 30 les valeurs en cours qui lui sont propres. De même, les circuits séquenceurs 60 fonctionnent alors en partage de temps entre les diverses pluralités, c'est-à-dire entre les divers individus. Précisément, pour un fonctionnement avec simultanéité des phases actives de mise en pression et de mesure ultérieures de plusieurs des brassards 9 ou auto-tensiomètres, l'unité centrale 10 fonctionne en exécutant, de façon entrelacée, les tâches concurrentes dans des tranches de temps respectivement et successivement allouées, une à une, à ces tâches. Chaque tranche de temps peut être prévue pour traiter toute la tâche considérée et ensuite libérer l'unité centrale 10 pour une tâche d'un autre ensemble de brassards 9- Il s'agit alors d'un fonctionnement asynchrone.

De façon préférable, toutefois, chaque tâche qui requiert la disponibilité de l'unité centrale 10 ne se voit allouer qu'une tranche temporellement de durée fixe, par exemple limitée à quelques millisecondes. Si la tâche n'est pas terminée dans ce délai, ce qui est le cas pour la surveillance de l'apparition de la recirculation artérielle, une temporisation commande que le contexte de cette tâche soit rangé en mémoire juste avant la fin de la tranche temporelle pour être repris lorsque cette tâche obtiendra à nouveau l'accès à l'unité centrale 10, après que des tâches liées à d'autres ensembles de brassards 9 ont été de même traitées. Outre les mesures de pression, cette mémorisation cyclique concerne aussi les circuits séquenceurs 60, qui peuvent ainsi, pour chaque ensemble de brassards 9, être reconfigurés dans leur état précédent. La réallocation des tranches temporelles peut s'effectuer selon un cycle de période prédéterminée, c'est-à-dire que chaque ensemble de brassards 9 dispose d'une tranche de temps particulière dans une trame temporelle. En variante, chaque ensemble actif de brassards 9, ou d'auto-tensiomètres, émet et maintient un état de requête en entrée de l'unité centrale 10, de sorte que la trame temporelle est réduite, de façon dynamique, aux seules tranches temporelles utiles, afin d'éviter les temps morts.

Les circuits de liaison 26 sont prévus pour traiter concurremment plusieurs liaisons, ici donc du type Internet, avec plusieurs circuits de raccordement 76 d'auto-tensiomètres (9) distants, c'est-à-dire échanger des paquets de données avec plusieurs équipements d'interface distants ayant chacun une adresse IP particulière. Compte tenu du temps de propagation dans l'Internet, bien supérieur à la durée d'une tranche temporelle ci-dessus, les circuits des liaison 26 fonctionnent de façon asynchrone par rapport à ces tranches, c'est-à-dire qu'ils comportent des mémoires, non représentées, d'interface interne avec l'unité centrale 10, pour en recevoir des données à émettre, cette émission pouvant ainsi intervenir à tout moment, et donc en particulier en dehors de la tranche temporelle considérée.

Le fonctionnement de l'unité centrale 7 va maintenant être expliqué plus en détails. L'unité centrale 7 a pour fonction de gérer les organes 2 à 5 du brassard 9, qui est alors un auto-tensiomètre. A cet effet, les circuits de liaison 26 et de raccordement 76 sont reliés par le réseau 100, qui, en variante, peut être un réseau local.

Les mémoires 72 et 73 contiennent respectivement des instructions de détermination de valeurs de mesure de pression artérielle et des instructions de détection de circulation artérielle, pour constituer, avec le bloc de calcul 71, des circuits de détermination de valeurs de mesure de pression artérielle, exploitant les grandeurs captées par le manomètre 3, et des circuits de détermination de détection de circulation artérielle, exploitant les grandeurs captées par le microphone 5. Deux sorties respectives de ces circuits sont reliées aux circuits de raccordement 76, pour transmettre les mesures, alors numérisées, à l'unité centrale 10. En variante, la fonction d'analyse sonore des circuits 25 est assurée par le bloc de calcul 71, de sorte que la décision de reconnaissance de bruit de circulation est prise localement, donc sans nécessité d'émission des signaux sonores correspondants.

La mémoire 72 est en outre reliée en entrée aux circuits de raccordement 76 pour recevoir les dites instructions à travers les circuits de raccordement réseau 76, en provenance de l'unité centrale 10 ou d'un serveur de mise en service.

L'unité centrale 7 est en outre ici prévue pour être télécommandée, par réception, à travers les circuits de raccordement 76, de commandes, émises par l'unité centrale 10, de lancement d'au moins trois cycles élémentaires correspondant respectivement aux dites mesures de pression.

Les circuits de raccordement 76 sont ici intégrés dans un poste téléphonique qui, dans cet exemple, est de type portable et prévu pour un réseau de radiotéléphonie, cellulaire ou par constellation de satellites. Le reste de l'unité centrale 7 pourrait être externe au poste et relié à celui-ci par un port d'entrée/sortie. Toutefois ici, toute l'unité centrale 7 est intégrée dans le poste. En particulier, le bloc de calcul 71 est agencé pour fonctionner en partage de temps entre des tâches de gestion de liaison téléphonique et des tâches liées au fonctionnement de l'équipement, précisément du brassard 9.

En variante, les divers circuits de l'unité centrale 10 nécessaires pour le calcul du dit rapport peuvent être intégrés dans le poste portable ci-dessus, qui représente ainsi un tel équipement complet. Les circuits de liaison réseau 26 et de raccordement réseau 76 sont fusionnés en un seul ensemble permettant de recevoir des télécommandes de déclenchement de mesures et/ou d'émettre le rapport calculé vers un centre de surveillance. Une telle forme de réalisation peut aussi se présenter sous la forme d'un bracelet-montre, le bracelet étant gonflable pour assurer la fonction de l'enveloppe 1.

Dans toutes les formes de réalisation, il peut aussi être prévu que l'unité centrale 10 mesure le rythme cardiaque, que ce soit lors de la décompression de l'enveloppe 1 ou hors pression, appliquée éventuellement sur le thorax. Les mesures sont transmises, à des moyens d'exploitation, de la même façon que pour les résultats issus des mesures de pression.

## Revendications

1. Procédé d'aide au diagnostic pour détecter une anomalie de pression artérielle chez un individu, procédé dans lequel, lors d'un relâchement progressif d'un étranglement initial d'un membre de l'individu ayant bloqué localement toute circulation artérielle, on détecte un instant de début de recirculation artérielle pour alors effectuer une mesure d'une pression instantanée d'étranglement, l'on effectue au moins trois dites mesures (31, 32 33) effectuées, à respectivement un premier et un second bras, et une mesure (33) à une jambe déterminée,
procédé **caractérisé par le fait qu'** on compare (40) les deux mesures (31, 32) de la paire pour sélectionner celle de valeur maximale, et on calcule (42) un rapport entre la mesure relative à la dite jambe et la mesure de valeur maximale de bras, pour ainsi obtenir un rapport de pressions artérielles systoliques.

2. Procédé selon la revendication 1, dans lequel la dite jambe déterminée est une première jambe examinée et on effectue en outre une dite mesure à une seconde jambe (34), on compare mutuellement les deux mesures des jambes (33, 34) afin de déterminer un écart entre les deux rapports respectifs de pressions systoliques.

3. Équipement pour la mise en oeuvre du procédé selon l'une des revendications 1 et 2, comportant une unité centrale (10) reliée à des moyens d'interface (20) agencés pour recevoir en entrée et fournir à l'unité centrale (10) une pluralité d'au moins trois signaux de pression artérielle, l'unité centrale (10) comprenant des moyens comparateurs (40) reliés aux moyens d'interface (20) et agencés pour comparer mutuellement deux signaux d'une paire constituée par des premier et deuxième dits signaux de pression, et sélectionner une valeur maximale de pression dans la dite paire de signaux de pression, et des moyens de calcul (42), reliés en aval des moyens d'interface (20) et en sortie des moyens comparateurs (40), agencés pour calculer un premier rapport de pressions artérielles systoliques entre une valeur d'un dit signal de pression, troisième, et la dite valeur maximale de pression sélectionnée dans la paire.

4. Équipement selon la revendication 3, dans lequel les moyens d'interface (20) sont agencés pour recevoir et fournir aussi au moins un quatrième signal de pression artérielle et les moyens de calcul (42) sont agencés pour calculer un deuxième rapport de pressions artérielles systoliques entre la valeur du quatrième signal de pression et la dite valeur maximale de pression sélectionnée dans la paire.

5. Équipement selon l'une des revendications 3 et 4, comprenant des circuits séquenceurs (60) agencés pour commander une succession de cycles élémentaires, d'étranglement initial et de relâchement de l'étranglement de membre, par respectivement une dite pluralité d'actionneurs (2) prévus pour provoquer les étranglements respectifs.

6. Équipement selon l'une des revendications 3 à 5, dans lequel les moyens d'interface (20) sont agencés pour fournir les signaux de pression de façon continue en phase d'étranglement et l'unité centrale (10) comporte des moyens de comparaison (62, 63), reliés aux moyens d'interface (20), agencés pour comparer les signaux de pression à une valeur de seuil maximal et pour, en cas de dépassement du seuil, engendrer des signaux respectifs, d'inhibition de commande de moyens d'étranglement de membre, et les transmettre aux moyens d'interface (20), agencés pour les retransmettre en sortie de l'équipement.

7. Équipement selon l'une des revendications 3 à 6, dans lequel les moyens d'interface (20) sont agencés pour fournir les signaux de pression de façon continue, en phase de relâchement de chaque étranglement de membre, ainsi qu'une dite pluralité de signaux associés de capteur sonore (5) représentant un bruit de circulation artérielle, et l'unité centrale (10) comporte des moyens d'analyse sonore (25) agencés pour comparer chaque signal de capteur sonore (5) à un seuil minimal et pour, en cas de dépassement du seuil, commander une mémorisation (40) d'une valeur instantanée du signal de pression associé et commander ultérieurement l'activation des moyens comparateurs (40) et des moyens de calcul (42) sur la base de la dite valeur de pression mémorisée (40).

8. Équipement selon l'une des revendications 3 à 7, dans lequel l'unité centrale (10) est agencée pour traiter concurremment plusieurs dites pluralités d'au moins trois signaux de pression artérielle, pour respectivement plusieurs individus.

9. Équipement selon l'une des revendications 3 à 8, dans lequel les moyens d'interface (20) comportent des circuits de liaison réseau (26) prévus pour être reliés à un réseau de transmission de données (100).

10. Équipement selon la revendication 9, dans lequel l'unité centrale (10) est agencée pour transmettre, aux circuits de liaison réseau (26), des informations contenant le dit premier rapport de pressions artérielles systoliques.

11. Équipement selon l'une des revendications 9 et 10, dans lequel l'unité centrale (10) est agencée pour transmettre, aux circuits de liaison réseau (26), des ordres de télécommande d'organes de tensiomètres de fourniture des dits premier, deuxième et troisième signaux de pression.

12. Équipement selon l'une des revendications 9 à 11, comprenant le dit réseau de transmission de données (100) et au moins un auto-tensiomètre (9) comportant un moyen (5, 71, 74) de détermination de détection de circulation artérielle et un moyen (3, 71, 73) de détermination de valeurs de mesure de pression artérielle relié en sortie à des circuits (76) de raccordement au dit réseau (100), agencés pour recevoir les mesures de pression artérielle et les transmettre sur le réseau (100).

13. Équipement selon la revendication 12, dans lequel les circuits de raccordement (76) sont intégrés dans un poste téléphonique.

## Claims

1. A diagnostic aid method for detecting abnormal blood pressure in a subject, wherein, while progressively releasing an initial constriction of a limb of the individual which has locally blocked off all arterial circulation, the time at which arterial recirculation starts is detected in order to then perform measurement of an instantaneous constriction pressure, at least three of said measurements (31, 32, 33) are performed at respectively a first and a second arm, and a measurement (33) at a determined leg, the method being **characterised in that** comparison (40) is made of the two measurements (31, 32) of the pair in order to select the one having maximum value, and a ratio is calculated (42) between measurement relative to said leg and measurement of maximum arm value, in order to obtain a ratio of systolic arterial pressures.

2. The method according to claim 1, in which the said determined leg is a first leg in examined and additionally said measurement is performed on a second leg (34), the two leg measurements (33, 34) are mutually compared in order to determine a deviation between the two respective systolic pressure ratios.

3. Equipment for carrying out the method according to one of claims 1 and 2, comprising a central processing unit (10) connected to interface means (20) arranged to receive as an input and supply to the central processing unit (10) a plurality of at least three arterial pressure signals, the central processing unit (10) comprising comparator means (40) connected to the interface means (20) and arranged for mutually comparing two signals of a pair constituted by the first and second said pressure signals, and selecting a maximum pressure value within the said pair of pressure signals, and calculating means (42), connected downstream of said interface means (20) and at the output of the comparator means (40), arranged for calculating a first systolic arterial pressure ratio between a value of a said pressure signal, third, and the said maximum pressure value selected within the pair.

4. The equipment according to claim 3, in which the interface means (20) are arranged to further receive and supply at least a fourth arterial pressure signal and the calculating means (42) are arranged to calculate a second systolic arterial pressure ratio between the value of the fourth pressure signal and the said maximum value of pressure, selected within the pair.

5. The equipment according to one of claims 3 and 4, comprising sequencing circuits (60) arranged for controlling a succession of individual cycles, of the initial constriction and release of member constriction, by, respectively, one of said plurality of actuators (2) provided for bringing about the respective constrictions.

6. The equipment according to one of claims 3-5, in which the interface means (20) are arranged for supplying pressure signals in continuous fashion during a constriction phase and the central processing unit (10) comprises comparison means (62, 63), connected to the interface means (20), arranged for comparing the pressure signals with a maximum threshold value and for, where said threshold is exceeded, generating respective signals for inhibiting member constriction means control, and transmitting them to the interface means (20), arranged for retransmitting them as an output from the equipment.

7. The equipment according to one of claims 3-6, in which the interface means (20) are arranged for supplying pressure signals in continuous fashion, during a release phase of each member constriction, together with said plurality of associated audible sensor signals (5) representing arterial circulation noise, and the central processing unit (10) comprises sound analysis means (25) arranged for comparing each audible sensor signal (5) with a minimum threshold and for, when said threshold is exceeded, controlling storage (40) of an associated pressure signal instantaneous value and subsequently controlling activation of the comparator means (40) and calculating means (42) on the basis of said stored pressure value (40).

8. The equipment according to one of claims 3-7, in which the central31.24 processing unit (10) is arranged for simultaneously processing several said pluralities of a least three arterial pressure signals, for, respectively, several individuals.

9. The equipment according to one of claims 3-8, in which the interface means (20) include network link circuits (26) provided for connection to a data transmission network (100).

10. The equipment according to claim 9, in which the central processing unit (10) is arranged for transmitting, to the network link circuits (26), information containing the said first systolic arterial pressure ratio.

11. The equipment according to one of claims 9 and 10, in which the central processing unit (10) is arranged to transmit, to the network link circuits (26), remoter control commands for BP measurement instrument units to supply the said first, second and third pressure signals.

12. The equipment according to one of claims 9-11, comprising the said data transmission network (100) and at least one self-operating BP measurement instrument (9) comprising a means (5, 71, 74) for determining detection of arterial circulation and a means (3, 71, 73) for determining arterial pressure measurement values connected at the output thereof to circuits (76) for connection to said network (100), arranged for receiving arterial pressure measurements and transmitting them over said network (100).

13. The equipment according to claim 12, in which the connections circuits (76) are integrated into a telephone set.

## Patentansprüche

1. Diagnosehilfsverfahren zum Erfassen einer Blutdruckanomalie bei einem Menschen, wobei man bei einem allmählichen Nachlassen eines ursprünglichen Drosselns eines Glieds des Menschen, das lokal die ganze Blutzirkulation blockiert hat, einen Anfangsaugenblick der Blutneuzirkulation erfasst, um dann eine Messung eines Drosselungssofortdrucks durchzuführen, man mindestens dre Messungen (31, 32, 33) jeweils an einem ersten und einem zweiten Arm und eine Messung (33) an einem bestimmten Bein durchführt, **dadurch gekennzeichnet, dass** man die zwei Werte (31, 32) des ersten Paars vergleicht (40), um den Höchstwert auszuwählen, und man ein Verhältnis zwischen der relativen Messung an dem Bein und der Armhöchstwertmessung berechnet (42), um **dadurch** ein Verhältnis systolischer Blutdrucke zu erzielen.

2. Verfahren nach Anspruch 1, bei dem das bestimmte Bein ein erstes untersuchtes Bein ist, und man ferner eine Messung an einem zweiten Bein (34) ausführt, man die zwei Messungen (33, 34) der Beine miteinander vergleicht, um einen Unterschied zwischen den zwei jeweiligen Verhältnissen systolischer Drucke zu bestimmen.

3. Ausstattung zum Umsetzen des Verfahrens nach einem der Ansprüche 1 und 2, das eine Zentraleinheit (10) aufweist, die mit Schnittstellenmitteln (20) verbunden ist, die eingerichtet sind, um einen Eingang zu empfangen und der Zentraleinheit (10) mehrere von mindestens drei Blutdrucksignalen zu liefern, wobei die Zentraleinheit (10) Vergleichsmittel (40) aufweist, die mit den Schnittstellenmitteln (20) verbunden und eingerichtet sind, um miteinander zwei Signale eines Paars zu vergleichen, das aus dem ersten und dem zweiten Drucksignal besteht, und einen Druckhöchstwert in dem Paar Drucksignale auszuwählen, und Rechenmittel (42), die stromabwärts der Schnittstellenmittel (20) und am Ausgang der Vergleichsmittel (40) verbunden sind, die eingerichtet sind, um ein erstes Verhältnis systolischer Blutdrucke zwischen einem dritten Drucksignal und dem aus dem Paar ausgewählten Druckhöchstwert zu berechnen.

4. Ausstattung nach Anspruch 3, bei der die Schnittsteilenmittel (20) eingerichtet sind, um auch mindestens ein viertes Blutdrucksignal zu empfangen und zu liefern, und wobei die Rechenmittel (42) eingerichtet sind, um ein zweites Verhältnis systolischer Blutdrücke zwischen dem Wert des vierten Drucksignals und dem Druckhöchstwert, der aus dem Paar ausgewählt wird, zu berechnen.

5. Ausstattung nach einem der Ansprüche 3 und 4, die Folgeschaltungen (60) aufweist, die eingerichtet sind, um eine Abfolge elementarer Zyklen des ursprünglichen Drosselns und Freigebens des Drosselns eines Glieds durch jeweils mehrere Stellantriebe (2), die vorgesehen sind, um die jeweiligen Drosselungen zu bewirken, zu steuern.

6. Ausstattung nach einem der Ansprüche 3 bis 5, bei der die Schnittstellenmittel (20) eingerichtet sind, um die Drucksignale ununterbrochen in Drosselphase zu liefern, und wobei die Zentraleinheit (10) Vergleichsmittel (62, 63) aufweist, die mit den Schnittstellenmitteln (20) verbunden sind, die eingerichtet sind, um die Drucksignale mit einem maximalen Schwellenwert zu vergleichen und im Fall des Überschreitens des Schwellenwerts jeweilige Signale zum Inhibieren der Steuerung der Drosselmittel des Glieds zu erzeugen und sie an die Schnittstellenmittel (20) zu übertragen, die eingerichtet sind, um sie zum Ausgang der Ausstattung weiter zu übertragen.

7. Ausstattung nach einem der Ansprüche 3 bis 6, bei der die Schnittstellenmittel (20) eingerichtet sind, um die Drucksignale ununterbrochen in Freigabephase jeder Drosselung eines Glieds zu liefern, sowie mehrere entsprechende Signale von Tonsensoren (5), die ein Blutzirkulationsgeräusch darstellen, wobei die Zentraleinheit: (10) Mittel zur Tonanalyse (25) aufweist, die eingerichtet sind, um jedes Tonsensorsignal (5) mit einem Mindestschwellenwert zu vergleichen und bei Überschreiten des Schwellenwerts ein Speichern (40) eines Sofortwerts des entsprechenden Drucksignals zu steuern und später das Aktivieren der Vergleichsmittel (40) und Rechenmittel (42) auf der Basis des gespeicherten Druckwerts (40) zu steuern.

8. Ausstattung nach einem der Ansprüche 3 bis 7, bei der die Zentraleinheit (10) eingerichtet ist, um gleichzeitig mehrere der Vielzahlen von mindestens drei Blutdrucksignalen für jeweils mehrere Personen zu verarbeiten.

9. Ausstattung nach einem der Ansprüche 3 bis 8, bei der die Schnittstellenmittel (20) Netzwerkverbindungsschaltungen (26) aufweisen, die vorgesehen sind, um mit einem Datenübertragungsnetzwerk (100) verbunden zu sein.

10. Ausstattung nach Anspruch 9, bei der die Zentraleinheit 10 eingerichtet ist, um an die Netzverbindungsschaltungen (26) Informationen zu übertragen, die das erste Verhältnis systolischer Blutdrucke enthalten.

11. Ausstattung nach einem der Ansprüche 9 und 10, bei der die Zentraleinheit (10) eingerichtet ist, um an die Netzwerkverbindungsschaltungen (26) Fernbedienungsbefehle von Druckmesselementen zum Liefern des ersten, zweiten und dritten Drucksignals zu übertragen.

12. Ausstattung nach einem der Ansprüche 9 bis 11, die das Datenübertragungsnetzwerk (100) und mindestens einen selbsttätige Druckmesser (9), der ein Mittel (5, 71, 74) zum Bestimmen des Blutzirkulationserfassens und ein Mittel (3, 71, 73) zum Bestimmen von Blutdruckmesswerten, die am Ausgang an Anschlussschaltungen (76) mit dem Netzwerk (100) angeschlossen sind, aufweist, die eingerichtet sind, um die Blutdruckmessungen zu empfangen und sie an das Netzwerk (100) zu übertragen.

13. Ausstattung nach Anspruch 12, bei der die Anschlussschaltungen (76) in ein Telefon integriert sind.
